# EUROPEAN PATENT APPLICATION

(11) **EP 1 757 310 A1**
(43) Date of publication of application: **28.02.2007**
(21) Application number: 05741406.2
(22) Date of filing: 19.05.2005
(51) Int. Cl.: A61K 47/38, A61K 9/48, A61K 47/32, A61K 47/34, A61J 3/07

(54) **SURFACE-MODIFIED AND SOLUBILITY-IMPROVED HARD CAPSULE**

(30) Priority: 24.05.2004 JP 2004153935
(71) Applicant: Qualicaps Co., Ltd., Nara 639-1032 (JP)
(72) Inventor: NAGATA, Shunji, 7370112 (JP); SAKUMA, Satoshi, 5200802 (JP)
(74) Representative: Teipel, Stephan
(86) International application number: PCT/JP2005/009135
(87) International publication number: WO 2005/113010

(57) **Abstract**

A hard capsule comprising a water-soluble cellulose derivative as a base material, **characterized in that** the water-soluble cellulose derivative is formulated with one or two or more compound(s) selected from polyvinylpyrrolidones, copolymers of vinylpyrrolidone with vinyl acetate, and polyethylene glycols having a weight-average molecular weight of 400 to 100,000. The hard capsule is easy to detach the cap part from the body part and facilitates the contents to be filled therein.

## Description

### TECHNICAL FIELD

The present invention relates to a surface-modified and solubility-improved hard capsule and a process for producing the same, and to a hard-capsule preparation.

### BACKGROUND ART

Capsules are available in soft and hard capsules, and the hard capsules are roughly grouped into gastric-coated hard capsules, which dissolve rapidly in the stomach, and enteric-coated hard capsules, which dissolve in the intestine but not in the stomach. Such gastric-coated hard capsules comprise a water-soluble cellulose derivative, such as hydroxypropyl methylcellulose (HPMC) and hydroxypropyl cellulose (HPC), which are used as a base material (Patent Documents 1 and 2).

In the meantime, hard capsule preparations are generally produced by detaching a capsule into its cap and body parts, and filling contents, such as medicinal drugs and healthy food materials, into the body part, followed by engagement of the body with the cap parts.

However, the hard capsules, as produced with use of the above-described water-soluble cellulose derivatives as a base material, often tend to develop irregularities on the surface, leading to difficulties in smoothened detachment into the cap and the body parts, and this causes troubles in filling contents into hard capsules.
Patent Document 1: Japanese Patent Application Laid-Open No. 3-279325
Patent Document 2: Japanese Patent Application Laid-Open No. 2000-136126

### DISCLOSURE OF THE INVENTION

### Problems to be Solved by the Invention

The present invention has as its object to provide hard capsules which are easy to get each of the cap and body parts detachably engaged with the other and also facilitate contents to be filled therein, and also a process for producing the same. Another object of the present invention is to provide solubility-improved hard capsules. Further another object of the present invention is to provide hard capsule preparations which comprise the hard capsule having contents filled therein.

### Means for Solving the Problems

The present inventors, with a specific view to attaining the above-described objects, conducted intensive studies, and as a result, found that formulation of the base material of a water-soluble cellulose derivative with one or two or more compound(s) selected from polyvinylpyrrolidones, copolymers of vinylpyrrolidone with vinyl acetate, and polyethylene glycols can produce hard capsules with easiness to get its cap part detachably engaged with its body part and that such capsule can facilitate the contents-filling operations to be carried out. The present inventors have also found that the above-mentioned hard capsules show improved solubility. The above-described findings, followed by further repeated investigation, culminated into completion of the present invention.

Namely, the present invention relates to:
(1) a hard capsule comprising a water-soluble cellulose derivative as a base material, characterized in that the water-soluble cellulose derivative is formulated with one or two or more compound(s) selected from polyvinylpyrrolidones, copolymers of vinylpyrrolidone with vinyl acetate, and polyethylene glycols having a weight-average molecular weight of 400 to 100,000 ;
(2) the hard capsule according to the above (1), wherein the water-soluble cellulose derivative is hydroxypropyl methylcellulose or hydroxypropyl cellulose;
(3) the hard capsule according to the above (1), wherein the water-soluble cellulose derivative is hydroxypropyl methylcellulose;
(4) the hard capsule according to the above (1), wherein the water-soluble cellulose derivative is formulated with one or two or more compound (s) selected from polyvinylpyrrolidones , copolymers of vinylpyrrolidone with vinyl acetate, and polyethylene glycols having a weight-average molecular weight of 400 to 100,000 at ratios of 0.5 to 20% by weight relative to 100% by weight of the water-soluble cellulose derivative;
(5) the hard capsule according to the above (1), wherein the water-soluble cellulose derivative is formulated with one or two or more compound (s) selected from polyvinylpyrrolidones , copolymers of vinylpyrrolidone with vinyl acetate, and polyethylene glycols at ratios of 1 to 15% by weight relative to 100% by weight of the water-soluble cellulose derivative;
(6) the hard capsule according to the above (1), wherein the weight-average molecular weight of the polyvinylpyrrolidone is 5,000 to 2,000,000;
(7) the hard capsule according to the above (1), wherein the copolymer of vinylpyrrolidone with vinyl acetate is copolyvidone;
(8) the hard capsule according to any one of the above (1) to (7), wherein the hard capsule is provided with improved detachability of the cap part from the body part;
(9) the hard capsule according to any one of the above (1) to (8), wherein the hard capsule shows improved solubility;
(10) a hard capsule preparation, wherein the hard capsule according to any one of the above (1) to (9) has contents filled therein;
(11) the hard capsule preparation according to the above (10), wherein the contents are medicinal drugs;
(12) a process for producing a hard capsule, which comprises forming the capsule by the dipping method with use of an aqueous solution containing (i) a water-soluble cellulose derivative, (ii) one or two or more compound(s) selected from polyvinylpyrrolidones, copolymers of vinylpyrrolidone with vinyl acetate, and polyethylene glycols having a weight-average molecular weight of 400 to 100,000, and (iii) a gelling agent; and
(13) the process according to the above (12), wherein the water-soluble cellulose derivative is hydroxypropyl methylcellulose or hydroxypropyl cellulose.

### EFFECTS OF THE INVENTION

The present invention provides hard capsules which are easy to get each of the cap and body parts detachably engaged with the other. Also, the present invention provides hard capsules with improved processability on the occasion of filling of the contents. Furthermore, the present invention provides hard capsules with improved solubility. In addition, there is offered the advantage that the hard capsules have a smooth surface and show an improved luster.

### BRIEF EXPLANATION OF THE DRAWING

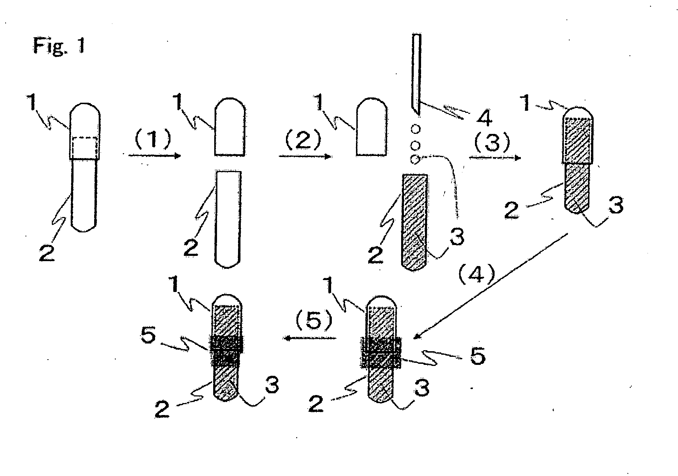

Fig. 1 is a schematic view showing the production steps for the hard capsule preparations according to the present invention, wherein the step (1) is a step of detaching the cap into the body and cap parts; the step (2) is a step of filling contents into the body part; the step (3) is a step of engaging the body part having the contents with the cap part; the step (4) is a step of sealing with a sealant the engaged body and cap parts to a hard capsule ; and the step (5) is a step of drying the sealed hard capsule, provided however that in the present invention, the steps (4) and (5) are not essentially required.

### EXPLANATORY REMARKS ON THE NUMERALS

1: Cap part of the hard capsule
2: Body part of the hard capsule
3: Contents
4: Filling tube or pipe
5: Sealing unit (band seal)

### BEST MODE FOR CARRYING OUT THE INVENTION

The present invention relates to a hard capsule comprising a water-soluble cellulose derivative as a base material, characterized in that the water-soluble cellulose derivative is formulated with one or two or more compound(s) selected from polyvinylpyrrolidones, copolymers of vinylpyrrolidone with vinyl acetate, and polyethylene glycols having a weight-average molecular weight of 400 to 100,000.

The water-soluble cellulose derivatives used as a base material in the present invention include , for example, cellulose ethers substituted with alkyl groups, particularly C₁ to C₄ lower alkyl groups and/or hydroxyalkyl groups, especially C₁ to C₄ hydroxy-lower alkyl groups, and as their typical examples, there may be mentioned hydroxypropyl methylcellulose (HPMC), hydroxyethyl cellulose, hydroxypropyl cellulose (HPC), hydroxyethyl methylcellulose, and the like. Among them, hydroxypropyl methylcellulose and hydroxypropylcellulose are especially preferable, and hydroxypropyl methylcellulose is more preferable.

The water-soluble cellulose derivatives are formulated with one or two or more compound(s) selected from polyvinylpyrrolidones, copolymers of vinylpyrrolidone with vinyl acetate, and polyethylene glycols having a weight-average molecular weight of 400 to 100,000 at ratios of, usually 0.5 to 20% by weight, preferably 0.5 to 17.5% by weight, and more preferably 1 to 15% by weight, relative to 100 parts by weight of the water-soluble cellulose derivative as a capsule base material. When the formulation ratio falls out of the above-described range of 0.5 to 20% by weight, the capsule parts may be difficult to be detachably engaged.

The polyvinylpyrrolidones to be formulated into the water-soluble cellulose derivative according to the present invention include, for example, povidone (compound No. 7783 in Merck Index, 13th Ed.). In the present invention, the commercially available products may be used as the polyvinylpyrrolidone, and examples thereof include PVPK-25, PVPK-30, and PVPK-90 (manufactured by Wako Pure Chemical Industries Co., Ltd.), Kollidon (manufactured by BASF Japan Ltd.), Plasdon (manufactured by ISP Japan Ltd.), and the like. The polyvinylpyrrolidone shows a weight-average molecular weight of normally 5, 000 to 2, 000, 000, preferably 25,000 to 1, 200, 000. When the weight-average molecular weight falls out of the range of 5,000 to 2,000,000, the capsule parts may be difficult to be detachably engaged.

The copolymers of vinylpyrrolidone with vinyl acetate to be formulated with the water-soluble cellulose derivative include, for example, copolyvidone (a copolymer of N-vinyl-2-pyrrolidone with vinyl acetate at a mass ratio of 60:40) described in Standard for Medical Additives (2003) and the like. Examples of the commercially available products include Plasdon SR, Plasdon S-630, and the like. The above-described copolyvidone shows a weight-average molecular weight of normally 5,000 to 1,000,000, preferably 10,000 to 100,000. When the weight-average molecular weight falls out of the range of 5,000 to 1,000,000, the capsule parts maybe difficult to be detachably engaged.

The polyethylene glycol to be formulated into the water-soluble cellulose derivative in the present invention shows a weight-average molecular weight of 400 to 100,000, more preferably 1,000 to 10 , 000 , and most preferably 1,200 to 9 , 500 . When the weight-average molecular weight falls out of the range of 400 to 100,000, the capsule parts may be difficult to be detachably engaged. Especially when the weight-average molecular weight exceeds 100,000, the capsule parts may be particularly difficult to be detachably engaged.

In the present invention, additives other than the polyvinylpyrrolidones, copolymers of vinylpyrrolidone with vinyl acetate, and polyethylene glycols having a weight-average molecular weight of 400 to 100,000 may be optionally formulated to the water-soluble cellulose derivative. Examples of the additives include gelling agents, plasticizers, gelling auxiliary agents, coloring matters, pigments, sugars, and the like.

As the gelling agents, there may be mentioned, for example, carrageenan, tamarind seed polysaccharide, pectin, curdlan, gelatin, furcellaran, agar, gellan gum, and the like, and carrageenan is particularly preferable.

The formulation ratio of the gelling agent is normally 0.1 to 3.0% by weight, preferably 0.25 to 2.5% by weight, and more preferably 0.5 to 2.0% by weight, to the capsule base material. When the formulation ratio of the gelling agent is less than 0.1% by weight, the formation of capsules may be difficult, whereas in the case of the formulation ratio in excess of 3.0% by weight, the resulting capsules may show deteriorated solubility.

Examples of the plasticizers include triethyl citrate, triacetin, Tween 80 (registered trademark) and the like, but the plasticizer may not necessarily be used.

As the gelling auxiliary agents, there may be mentioned, for example, the known ones and more specifically, potassium chloride, ammonium chloride, ammonium acetate, and calcium chloride.

As the coloring matters, pigments, and sugars, there may be mentioned individually those known in the art.

The hard capsules of the present invention are produced according to the known processes for producing hard capsules. For example, the capsules are produced by forming capsules by the dipping method with use of an aqueous solution (capsule-forming solution) containing (i) a water-soluble cellulose derivative, (ii) one or two or more compound(s) selected from polyvinylpyrrolidones, copolymers of vinylpyrrolidone with vinyl acetate, and polyethylene glycols having a weight-average molecular weight of 400 to 100,000, and (iii) miscellaneous additives (in particular, gelling agent). The hard capsules of the present invention can also be produced by the injection molding process.

More specifically described below is the production of the hard capsules according to the present invention, while taking the dipping method for an example.

The hard capsules of the present invention to be produced by the dipping method are formed by preparing a capsule-forming solution (the step of preparing a capsule-forming solution), dipping a pin for forming capsules in the solution, and following subsequently the known procedures to form capsules (the step of forming capsules).

Then, the step of preparing a capsule-forming solution and the step of forming capsules are to be described below.

In the step of preparing a capsule-forming solution, a capsule-forming solution is prepared.

The above-described capsule-forming solution is normally prepared by dissolving in water (i) a water-soluble cellulose derivative, (ii) one or two or more compound(s) selected from polyvinylpyrrolidones, copolymers of vinylpyrrolidone with vinyl acetate, and polyethylene glycols having a weight-average molecular weight of 400 to 100,000, and (iii) miscellaneous additives (in particular, gelling agent). The order of dissolution is not particularly limited, and either the water-soluble cellulose derivative, or the polyvinylpyrrolidone, copolymer of vinylpyrrolidone with vinyl acetate, or polyethylene glycol having a weight-average molecular weight of 400 to 100,000 may be dissolved first. The dissolution temperature is also not particularly limited, but is normally 40 to 100°C, preferably 50 to 95°C.

In the step of forming capsules, normally, pins for forming capsules are dipped in the capsule-forming solution and drawn out from the solution, and the capsule-forming solution adhered onto the pins are gelled and dried to thereby form the body and cap parts of the hard capsule.

The temperature at which the pins for forming capsules are dipped is not particularly limited, but is normally from 30 to 80°C, preferably from 40 to 60°C. The gelling after the pins are drawn out is preferably effected by being allowed to cool, but after gelling, drying by heating may be performed at 40 to 80°C.

In the said step of forming capsules, the body and cap parts of the hard capsule are obtainable separately by using the individually different-sized pins for forming capsules, and each of such body and cap parts is engaged with the other to form the hard capsule of the present invention.

Hard capsules are available in sizes, such as Nos. 00, 0, 1, 2, 3, 4 and 5 and the like but the hard capsules of the present invention may be of any sizes. These sizes are adjustable by suitably selecting the pin size in the step of forming capsules.

The hard-capsule preparations according to the present invention are produced by filling contents into the hard capsule.

Examples of such contents include drugs, food materials, and the like, and such contents may be in any forms of powders, granules, oils, gels, solutions and the like.

As the drugs , for example, use is made of one or two or more drug(s) selected from nourishment tonics, antipyretic/analgesic/antiinflammatory drugs, psychotropic drugs, anti-anxiety drugs, antidepressant drugs, hypnotic/sedative drugs, antispasmodic drugs, drugs acting on the central nervous system, cerebral metabolism improvers, cerebral circulation improvers, antiepileptic drugs, sympathetic nerve stimulants, digestives, antacids, antiulcer drugs, antitussive/expectorant drugs, antiemetic drugs, respiration promoters, bronchodilators, antiallergic drugs, drugs for dentistry and oral cavity, antihistaminic drugs, cardiotonics drugs, antiarrhythmic drugs, diuretic drugs, antihypertensive drugs, vasoconstrictors, coronary vasodilators, peripheral vasodilators, antihyperlipidemic drugs, cholagogues, antibiotics, chemotherapeutic drugs, antidiabetic drugs, antiosteoporotic drugs, antirheumatic drugs, skeletal muscle relaxants, spasmolytic drugs, hormone preparations, alkaloid narcotics, sulfa drugs, anti-gout drugs, anticoagulant drugs, antineoplastic drugs and the like.

Examples of the nourishment tonics include vitamins, such as vitamin A, vitamin D, vitamin E (such as d-α-tocopherol acetate), vitamin B₁ (such as dibenzoylthiamine and fursulthiamine hydrochloride), vitamin B₂ (such as riboflavin butyrate), vitamin B₆ (such as pyridoxine hydrochloride), vitamin C (such as ascorbic acid and L-sodium ascorbate), and vitamin B₁₂ (such as hydroxocobalamine acetate and cyanocobalamine), minerals (such as calcium, magnesium, and iron), proteins, amino acids, oligosaccharides, herbal medicines and the like.

Examples of the antipyretic/analgesic/antiinflammatory drugs include aspirin, acetaminophen, ethenzamide, ibuprofen, diphenhydramine hydrochloride, dl-chlorophenylamine maleate, dihydrocodeine phosphate, noscapine, methylephedrine hydrochloride, phenylpropanolamine hydrochloride, caffeine, anhydrous caffeine, serrapeptase, lysozyme hydrochloride, tolfenamic acid, mefenamic acid, diclofenac sodium, flufenamic acid, salicylamide, aminopyrine, ketoprofen, indomethacin, bucolome, pentazocine, and the like.

Examples of the psycotropic drugs include chlorpromazine, reserpine, and the like.

Examples of the antianxiety drugs include alprazolam, chlordiazepoxide, diazepam, and the like.

Examples of the antidepressant drugs include imipramine, maprotiline hydrochloride, amphetamine, and the like.

Examples of the hypnotic/sedative drugs include estazolam, nitrazepam, diazepam, perlapine, phenobarbital sodium, and the like.

Examples of the antispasmodic drugs include scopolamine hydrobromide, diphenhydramine hydrochloride, papaverine hydrochloride, and the like.

Examples of the drugs acting on the central nervous system include citicoline, and the like.

Examples of the cerebral metabolism improvers include meclofenoxate hydrochloride and the like.

Examples of the cerebral circulation improvers include vinpocetine and the like.

Examples of the antiepileptic drugs include phenytoin, carbamazepine and the like.

Examples of the sympathetic nerve stimulant drugs include isoproterenol hydrochloride and the like.

Examples of the digestives include stomach digestives, such as diastase, saccharated pepsin, scopolia extract, cellulase AP3, lipase AP, and cinnamon oil, and intestinal regulators such as berberine hydrochloride, resistant lactic bacteria, and lactobacillus bifidus, and the like.

Examples of the antacids include magnesium carbonate, sodium bicarbonate, magnesium aluminate metasilicate, synthetic hydrotalcite, precipitated calcium carbonate, magnesium oxide, and the like.

Examples of the antiulcer drugs include lansoprazole, omeprazole, rabeprazole, famotidine, cimetidine, ranitidine hydrochloride, and the like.

Examples of the antitussive/expectorant drugs include cloperastine hydrochloride, dextrometrofan hydrobromide, theophylline, potassium guaiacolsulfonate, guaifenesin, codeine phosphate, and the like.

Examples of the antiemetic drugs include diphenidol hydrochloride, metoclopramide, and the like.

Examples of the respiration promoters include levallorphan tartarate, and the like.

Examples of the bronchodilators include theophylline, sulbutamol sulfate, and the like.

Examples of the antiallergic drugs include amlexanox, seratrodast, and the like.

Examples of the drugs for dentistry and oral cavity include oxytetracycline, triamcinolone acetonide, chlorhexidine hydrochloride, lidocaine, and the like.

Examples of the antihistaminic drugs include diphenhydramine hydrochloride, promethazine, isothipendyl hydrochloride, dl-chlorphenylamine maleate, and the like.

Examples of the cardiotonics include caffeine, digoxin, and the like.

Examples of the antiarrhythmic drugs include procainamide hydrochloride, propranolol hydrochloride, pindolol, and the like.

Examples of the diuretic drugs include isosorbide, furosemide, hydrochlorothiazide, and the like.

Examples of the antihypertensive drugs include delapril hydrochloride, captopril, hydralazine hydrochloride, labetalol hydrochloride, manidipine hydrochloride, candesartan cilexetil, methyldopa, perindopril erbumine, and the like.

Examples of the vasoconstrictors include phenylephrine hydrochloride, and the like.

Examples of the coronary vasodilators include carbocromen hydrochloride, molsidomine, verapamil hydrochloride, and the like.

Examples of the peripheral vasodilators include cinnarizine, and the like.

Examples of the antihyperlipidemic drugs include cerivastatin sodium, simvastatin, pravastatin sodium, atorvastatin calcium hydrate, and the like.

Examples of the cholagogues include dehydrocholic acid, trepibutone, and the like.

Examples of the antibiotics include cephem antibiotics, such as cephalexin, cefaclor, amoxicillin, pivmecillinam hydrochloride, cefotiam hexetil hydrochloride, cefadroxil, cefixime, cefditoren pivoxil, cefteram pivoxil and cefpodoxime proxetil; synthetic antibacterial agents, such as ampicillin, ciclacillin, nalidixic acid and enoxacin; monobactam antibiotics, such as carumonam sodium; penem and carbapenem antibiotics, and the like.

Examples of the chemotherapeutic drugs include sulfamethizole, and the like.

Examples of the antidiabetic drugs include tolbutamide, voglibose, pioglitazone hydrochloride, glibenclamide, troglitazone, and the like.

Examples of the antiosteoporotic drugs include ipriflavone and the like.

Examples of the skeletal muscle relaxants include methocarbamol, and the like.

Examples of the spasmolytic drugs include meclizine hydrochloride, dimenhydrinate, and the like.

Examples of the antirheumatic drugs include methotrexate, bucillamine, and the like.

Examples of the hormone preparations include liothyronine sodium, dexamathasone sodium phosphate, prednisolone, oxendolone, leuprorelin acetate, and the like.

Examples of the alkaloid narcotics include opium, morphine hydrochloride, ipecacuanha, oxycodone hydrochloride, opium alkaloid hydrochloride, cocaine hydrochloride, and the like.

Examples of the sulfa drugs include sulfisomidine, sulfamethizole, and the like.

Examples of the anti-gout drugs include allopurinol, colchicine, and the like.

Examples of the anticoagulant drugs include dicumarol, and the like.

Examples of the anti-neoplastic drugs include 5-fluorouracil, uracil, mitomycin, and the like.

Examples of the food materials include garlic powder, royal jelly, soft-shelled turtle powder, chlorella, spirulina, β-carotene, calcium, oyster processed products, vitamin C, dietary fibers, yeast foods, lecithin processed products, *shiitake* mushroom processed products, gymnema, maidenhair leaf extract, chitin, chitosan, and the like.

The contents can be filled in the hard capsules, for example, by use of the *per* se known capsule-filling machines, such as full automatic capsule-filling machine(Model name : LIQFILsuper 80/150, manufactured by Shionogi Qualicaps Co., Ltd.) or a capsule-filling and sealing machine (Model name: LIQFILsuper FS, manufactured by Shionogi Qualicaps Co., Ltd.).

In the present invention, after filling of the contents in the body part of the hard capsule, a band-seal may be formed in the shape of a belt from the surface edge portion of the cap part to the body part, followed by fastening of the body and cap parts to each other to thereby carry out sealing of the hard capsule preparation.

Sealing of the hard capsule preparations can be conducted into practice by use of the per se known capsule filling machines, such as the above-mentioned capsule filling/sealing machine (Model name: HICAPSEAL 40/100, manufactured by Shionogi Qualicaps Co., Ltd.).

Fig. 1 is a schematic view showing a production process for hard capsule preparations as sealed with a band seal. In the step (1), the body and cap parts of hard capsule are detached from each other and in the step (2), the contents are filled into the body part. In the step (3), the cap part is engaged with the body part having the contents packed. In the step (4), the engaged hard capsule preparation is subjected to sealing with a seal material, and the sealed hard capsule preparation is dried in the step (5).

### Examples

Below described are the examples to illustrate the present invention, but the present invention is not understood to be limited to these examples. In the following Examples, "%" means "% by weight", unless otherwise specified. Hard capsules of Size No. 3 are prepared in all of the Examples, but the present invention should not be understood to depend upon the size of the hard capsule.

### (Example 1)

Dissolved in water at approximately 80°C were 20% by weight of hydroxypropyl methylcellulose (tradename of Metolose, manufactured by Shin-Etsu Chemical Co., Ltd.) and 1% by weight of a polyvinylpyrrolidone (PVPK-90 with a weight-average molecular weight of 360,000, manufactured by Wako Pure Chemical Industries Co. , Ltd.), and the solution was warmed at about 55°C. To the solution were added 0.08% by weight of carrageenan and 0.08% by weight of potassium chloride, and water was added thereto to make a total amount of 100% by weight, followed by deaeration. A pin made of stainless-steel coated with lecithin (mold-releasing agent) was dipped into, and drawn out from, the solution at about 5°C, and dried at 25°C to 50°C to give a hard capsule (Size No. 3).

### (Example 2)

A hard capsule (Size No. 3) was produced in the same manner as described in Example 1, except that in place of the polyvinylpyrrolidone (PVPK-90 with a weight-average molecular weight of 360 , 000 , manufactured by Wako Pure Chemical Industries Co., Ltd.), a polyethylene glycol (PEG 6000 with a weight-average molecular weight of 7,300 to 9,300, manufactured by Wako Pure Chemical Industries Co., Ltd.) was used.

### (Example 3)

A hard capsule (Size No. 3) was produced in the same manner as described in Example 1, except that in place of polyvinylpyrrolidone (PVPK-90 with a weight-average molecular weight of 360, 000, manufactured by Wako Pure Chemical Industries Co., Ltd.), a polyethylene glycol (PEG 4000 with a weight-average molecular weight of 2,600 to 3,800, manufactured by Wako Pure Chemical Industries Co., Ltd.) was used.

### (Example 4)

A hard capsule (Size No. 3) was produced in the same manner as described in Example 1, except that in place of the polyvinylpyrrolidone (PVPK-90 with a weight-average molecular weight of 360,000, manufactured by Wako Pure Chemical Industries Co. , Ltd.), a polyethylene glycol (PEG 2000 with a weight-average molecular weight of 1,850 to 2,150, manufactured by Kanto Chemical Co. Inc.).

### (Example 5)

A hard capsule (Size No. 3) was produced in the same manner as described in Example 1, except that in place of the polyvinylpyrrolidone (PVPK-90 with a weight-average molecular weight of 360 , 000 , manufactured by Wako Pure Chemical Industries Co., Ltd.), copolyvidone (a copolymer of N-vinyl-2-pyrrolidone with vinyl acetate (weight ratio 60:40), having a weight-average molecular weight of about 50,000; tradename of Plasdon S-630; manufactured by ISP Japan Ltd.).

### (Example 6)

A hard capsule (Size No. 3) was produced in the same manner as described in Example 1, except that in place of the polyvinylpyrrolidone (PVP-K90 with a weight-average molecular weight of 360 , 000 , manufactured by Wako Pure Chemical Industries Co., Ltd.), 3% by weight of copolyvidone (a copolymer of N-vinyl-2-pyrrolidone with vinyl acetate at a weight ratio of 60:40, having a weight-average molecular weight of about 50,000; trade name of Plasdon S-630, manufactured by ISP Japan Ltd.) and 76.84 % by weight of water were used.

### (Comparative Example 1)

A hard capsule (Size No. 3) was produced in the same manner as in Example 1, except that without use of the polyvinylpyrrolidone (PVPK-90 with a weight-average molecular weight of 360,000, manufactured by Wako Pure Chemical Industries Co., Ltd.), 79.84% by weight of water was used.

### (Comparative Example 2)

A hard capsule (Size No. 3) was produced in the same manner as described in Example 1, except that in place of the polyvinylpyrrolidone (PVPK-907 with a weight-average molecular weight of 360,000, manufactured by Wako Pure Chemical Industries Co. , Ltd.), there were used 0.2% by weight of Aerosil and 79.64% by weight of water.

### (Comparative Example 3)

A hard capsule (Size No. 3) was produced in the same manner as described in Example 1, except that in place of the polyvinylpyrrolidone (PVP-K90 with a weight-average molecular weight of 360 , 000 , manufactured by Wako Pure Chemical Industries Co., Ltd.), a polyethylene glycol having a weight-average molecular weight of 200 , 000 (POLYOX N80, manufactured by Union Carbide Corp.) was used.

### (Test Example 1)

With 10 hard capsules as produced individually in Examples 1 to 6 and Comparative Examples 1 and 2 and by use of AUTOGRAPH (manufactured by Shimadzu Corporation), measurements were made of the force required to transform a temporary bonding between the cap and body parts of the capsule to the permanent bonding, whereby AUTOGRAH, with its 100N load cell, was operated to compress each capsule at a speed of 10 mm/minute until the length of bonding between the cap and body parts becomes to be 2.1 mm. Measurement results are tabulated in Table 1.

**Table 1**

| | Force (N) required for bonding ± standard deviation |
|---|---|
| Example 1 | 19.2±0.363 |
| Example 2 | 18.1±0.352 |
| Example 3 | 19.2±0.344 |
| Example 4 | 19.8±0.356 |
| Example 5 | 16.3±0.362 |
| Example 6 | 14.8±0.366 |
| Comparative Example 1 | 23.2±0.342 |
| Comparative Example 2 | 27.2±0.347 |
| Comparative Example 3 | 24.3±0.322 |

The measurement results tabulated above demonstrate that when polyvinylpyrrolidone or polyethylene glycol was added to the shells of the hydroxypropyl methylcellulose capsules, the resultant capsules show a lowered force required for bonding, as compared with those produced without addition of any additives (Comparative Example 1), facilitating each of the body and cap parts in hydroxypropyl methylcellulose capsules to be detachably engaged with the other. The capsules of Examples 2 to 4 as produced by addition of polyethylene glycols having lower weight-average molecular weights than the polyethylene glycol with a weight-average molecular weight of 200,000 as used in Comparative Example 3 showed a decreased force required for bonding, as compared with those of Comparative Example 3, and this suggested that the degree of easiness varies in detachable engagement of the cap with body parts, depending upon the weight-average molecular weight of the additives, particularly polyethylene glycols.

### (Example 7)

0.12% by weight of potassium chloride and 2% by weight of a copolymer of 1-vinyl-2-pyrrolidone with vinyl acetate (copolyvidone; product name of Plasdon S-630, manufactured by ISP Japan Ltd. ) were added to 78.6% by weight of water (purified water), followed by complete dissolution. The solution was warmed to 80°C on a water bath, and 18% by weight of hydroxypropyl methylcellulose (HPMC) and 1.2% by weight of titanium oxide were added, followed by adequate stirring to form a uniform dispersion (hereinafter referred to as "HPMC dispersion"). Separately, κ-carrageenan was added to water (purified water) and heated to 80 to 90°C on a water bath, under stirring, to prepare a 4% aqueous carrageenan solution, which was then added to the HPMC dispersion to the carrageenan concentration of 0.08% by weight. The mixture was transferred in a desiccator for reduced pressure, which was kept under reduced pressure at 55°C for not less than 1 hour to make degassing. Afterdegassing, the mixture was stored at 55°C for one day in an oven. The thus obtained HPMC dispersion was poured into a polyacrylate-made container previously warmed at 55°C, followed by immersion of a pin bar for capsule production. The pin bar was drawn out of the above-described dispersion, and dried under aeration at room temperature for 2 hours to give a hard capsule (Size No. 3).

### (Comparative Example 4)

A hard capsule (Size No. 3) was produced in the same manner as described in Example 7 , except that without use of the copolymer (copolyvidone) of 1-vinyl-2-pyrrolidone with vinyl acetate, hydroxypropyl methylcellulose was used at a ratio of 20% by weight.

### (Test Example 2)

### Elution test of the drug substances filled into the hard capsule

A 100 mg quantity of the composition comprising 20% by weight of acetaminophen, 70% by weight of lactose and 10% by weight of croscarmellose sodium was filled in each of the hard capsules as produced in Example 7 and Comparative Example 4, and a dissolution test was performed according to the paddle method (with a sinker) specified in the Japanese Pharmacopoeia 15th Ed. by using the Second Solution of the Japanese Pharmacopeia. The results are shown in Table 2.

**Table 2: Comparison of dissolution characteristics of drug substance as filled in hard capsules**

| Disintegration time, min. | Dissolution rate (%) | |
|---|---|---|
| | Hard capsule of Example 7 | Hard capsule of Comparative Example 4 |
| 0 | 0 | 0 |
| 5 | 0.3 | 1.4 |
| 10 | 18.8 | 14.9 |
| 15 | 72.5 | 33.0 |
| 20 | 87.9 | 47.5 |
| 30 | 96.4 | 73.8 |

As apparent from Table 2, the HPMC hard capsule obtained in Example 7 of the present invention showed a shorter disintegration time, as compared with the HPMC hard capsule of Comparative Example 4 and was found to be markedly improved in dissolution characteristics.

### INDUSTRIAL APPLICABILITY

The hard capsules according to the present invention are useful in the production of hard capsule preparations. In addition, the hard capsule preparations according to the present invention are useful for oral administration of drugs and food compositions.

## Claims

1. A hard capsule comprising a water-soluble cellulose derivative as a base material, **characterized in that** the water-soluble cellulose derivative is formulated with one or two or more compound(s) selected from polyvinylpyrrolidones, copolymers of vinylpyrrolidone with vinyl acetate, and polyethylene glycols having a weight-average molecular weight of 400 to 100,000.

2. The hard capsule according to claim 1, wherein the water-soluble cellulose derivative is hydroxypropyl methylcellulose or hydroxypropyl cellulose.

3. The hard capsule according to claim 1, wherein the water-soluble cellulose derivative is hydroxypropyl methylcellulose.

4. The hard capsule according to claim 1, wherein the water-soluble cellulose derivative is formulated with one or two or more compound(s) selected from polyvinylpyrrolidones, copolymers of vinylpyrrolidone with vinyl acetate, and polyethylene glycols having a weight-average molecular weight of 400 to 100,000 at ratios of 0.5 to 20% by weight relative to 100% by weight of the water-soluble cellulose derivative.

5. The hard capsule according to claim 1, wherein the water-soluble cellulose derivative is formulated with the one or two or more compound (s) selected from polyvinylpyrrolidones , copolymers of vinylpyrrolidone with vinyl acetate, and polyethylene glycols having a weight-average molecular weight of 400 to 100,000 at ratios of 1 to 15% by weight relative to 100% by weight of the water-soluble cellulose derivative.

6. The hard capsule according to claim 1, wherein the weight-average molecular weight of the polyvinylpyrrolidone is 5,000 to 2,000,000.

7. The hard capsule according to claim 1, wherein the copolymer of vinylpyrrolidone with vinyl acetate is copolyvidone.

8. The hard capsule according to any one of claims 1 to 7, wherein the hard capsule is provided with improved detachability of the cap part from the body part.

9. The hard capsule according to any one of Claims 1 to 8, wherein the hard capsule shows improved solubility.

10. A hard capsule preparation, wherein the hard capsule according to any one of claims 1 to 9 has contents filled therein.

11. The hard capsule preparation according to claim 10, wherein the contents are a medicinal drug.

12. A process for producing a hard capsule, which comprises forming the capsule by the dipping method with use of an aqueous solution containing (i) a water-soluble cellulose derivative, (ii) one or two or more compound(s) selected from polyvinylpyrrolidones, copolymers of vinylpyrrolidone with vinyl acetate, and polyethylene glycols having a weight-average molecular weight of 400 to 100,000, and (iii) a gelling agent.

13. The process according to claim 12, wherein the water-soluble cellulose derivative is hydroxypropyl methylcellulose or hydroxypropyl cellulose.
